# EUROPEAN PATENT SPECIFICATION

(11) **EP 3 337 821 B1**
(45) Date of publication and mention of the grant of the patent: **18.03.2020**
(21) Application number: 16756993.8
(22) Date of filing: 16.08.2016
(51) Int. Cl.: C07K 16/18, G01N 33/574, G01N 33/68

(54) **IMMUNOASSAY FOR COLLAGEN TYPE VIII SEQUENCES**
IMMUNOASSAY FÜR COLLAGEN-TYP-VIII-SEQUENZEN
DOSAGE IMMUNOLOGIQUE DE SÉQUENCES DE COLLAGÈNE DE TYPE VIII

(30) Priority: 18.08.2015 GB 201514658
(43) Date of publication of application: 27.06.2018
(73) Proprietor: Nordic Bioscience A/S, 2730 Herlev (DK)
(72) Inventor: HANSEN, Niels, Ulrik, Brandt, 2400 Copenhagen NV (DK); LEEMING, Diana, Julie, Oersnes, 3060 Espergaerde (DK); KARSDAL, Morten, Asser, 2100 København Ø (DK)
(74) Representative: Beck Greener LLP
(86) International application number: PCT/EP2016/069453
(87) International publication number: WO 2017/029300

(56) References cited:
- "Anti-Collagen VIII alpha 1 antibody ab58776 - Prodduct darasheet", Abcam Online Catalogue , 24 October 2016 (2016-10-24), XP002763305, Retrieved from the Internet: URL:http://www.abcam.com/Collagen-VIII-alp ha-1-antibody-ab58776.pdf [retrieved on 2016-10-24]
- KORSCHING E ET AL: "Generation of type VIII collagen-specific antibodies.", JOURNAL OF IMMUNOLOGICAL METHODS 15 DEC 1995, vol. 188, no. 1, 15 December 1995 (1995-12-15), pages 51-62, XP002763306, ISSN: 0022-1759
- MA, Z.-H.; MA, J.-H.; JIA, L.; ZHAO, Y.-F.: "Effect of enhanced expression of COL8A1 on lymphatic metastasis of hepatocellular carcinoma in mice", EXP. THER. MED., vol. 4, 2012, pages 621-626, XP002763307, cited in the application
- HANSEN NIELS ULRIK BRANDT ET AL: "Type VIII collagen is elevated in diseases associated with angiogenesis and vascular remodeling", CLINICAL BIOCHEMISTRY, ELSEVIER INC, US, CA, vol. 49, no. 12, 24 May 2016 (2016-05-24), pages 903-908, XP029668258, ISSN: 0009-9120, DOI: 10.1016/J.CLINBIOCHEM.2016.05.023

### SEQUENCE LISTING

<110> Nordic Bioscience A/S
<120> Immunoassay for Collagen Type VIII Sequences
<130> P19112WO
<150> GB1514658.2
   <151> 2015-08-18
<160> 1
<170> PatentIn version 3.5
<210> 1
   <211> 744
   <212> PRT
   <213> Homo sapiens
<400> 1
   Met Ala Val Leu Pro Gly Pro Leu Gln Leu Leu Gly Val Leu Leu Thr
   1               5                   10                  15
   Ile Ser Leu Ser Ser Ile Arg Leu Ile Gln Ala Gly Ala Tyr Tyr Gly
               20                  25                  30
   Ile Lys Pro Leu Pro Pro Gln Ile Pro Pro Gln Met Pro Pro Gln Ile
           35                  40                  45
   Pro Gln Tyr Gln Pro Leu Gly Gln Gln Val Pro His Met Pro Leu Ala
       50                  55                  60
   Lys Asp Gly Leu Ala Met Gly Lys Glu Met Pro His Leu Gln Tyr Gly
   65                  70                  75                  80
   Lys Glu Tyr Pro His Leu Pro Gln Tyr Met Lys Glu Ile Gln Pro Ala
                   85                  90                  95
   Pro Arg Met Gly Lys Glu Ala Val Pro Lys Lys Gly Lys Glu Ile Pro
               100                 105                 110
   Leu Ala Ser Leu Arg Gly Glu Gln Gly Pro Arg Gly Glu Pro Gly Pro
           115                 120                 125
   Arg Gly Pro Pro Gly Pro Pro Gly Leu Pro Gly His Gly Ile Pro Gly
       130                 135                 140
   Ile Lys Gly Lys Pro Gly Pro Gln Gly Tyr Pro Gly Val Gly Lys Pro
   145                 150                 155                 160
   Gly Met Pro Gly Met Pro Gly Lys Pro Gly Ala Met Gly Met Pro Gly
   165 170 175
   Ala Lys Gly Glu Ile Gly Gln Lys Gly Glu Ile Gly Pro Met Gly Ile
   180 185 190
   Pro Gly Pro Gln Gly Pro Pro Gly Pro His Gly Leu Pro Gly Ile Gly
   195 200 205
   Lys Pro Gly Gly Pro Gly Leu Pro Gly Gln Pro Gly Pro Lys Gly Asp
   210 215 220
   Arg Gly Pro Lys Gly Leu Pro Gly Pro Gln Gly Leu Arg Gly Pro Lys
   225 230 235 240
   Gly Asp Lys Gly Phe Gly Met Pro Gly Ala Pro Gly Val Lys Gly Pro
   245 250 255
   Pro Gly Met His Gly Pro Pro Gly Pro Val Gly Leu Pro Gly Val Gly
   260 265 270
   Lys Pro Gly Val Thr Gly Phe Pro Gly Pro Gln Gly Pro Leu Gly Lys
   275 280 285
   Pro Gly Ala Pro Gly Glu Pro Gly Pro Gln Gly Pro Ile Gly Val Pro
   290 295 300
   Gly Val Gln Gly Pro Pro Gly Ile Pro Gly Ile Gly Lys Pro Gly Gln
   305 310 315 320
   Asp Gly Ile Pro Gly Gln Pro Gly Phe Pro Gly Gly Lys Gly Glu Gln
   325 330 335
   Gly Leu Pro Gly Leu Pro Gly Pro Pro Gly Leu Pro Gly Ile Gly Lys
   340 345 350
   Pro Gly Phe Pro Gly Pro Lys Gly Asp Arg Gly Met Gly Gly Val Pro
   355 360 365
   Gly Ala Leu Gly Pro Arg Gly Glu Lys Gly Pro Ile Gly Ala Pro Gly
   370 375 380
   Ile Gly Gly Pro Pro Gly Glu Pro Gly Leu Pro Gly Ile Pro Gly Pro
   385 390 395 400
   Met Gly Pro Pro Gly Ala Ile Gly Phe Pro Gly Pro Lys Gly Glu Gly
   405 410 415
   Gly Ile Val Gly Pro Gln Gly Pro Pro Gly Pro Lys Gly Glu Pro Gly
               420                 425                 430
   Leu Gln Gly Phe Pro Gly Lys Pro Gly Phe Leu Gly Glu Val Gly Pro
           435                 440                 445
   Pro Gly Met Arg Gly Leu Pro Gly Pro Ile Gly Pro Lys Gly Glu Ala
       450                 455                 460
   Gly Gln Lys Gly Val Pro Gly Leu Pro Gly Val Pro Gly Leu Leu Gly
   465                 470                 475                 480
   Pro Lys Gly Glu Pro Gly Ile Pro Gly Asp Gln Gly Leu Gln Gly Pro
                   485                 490                 495
   Pro Gly Ile Pro Gly Ile Gly Gly Pro Ser Gly Pro Ile Gly Pro Pro
               500                 505                 510
   Gly Ile Pro Gly Pro Lys Gly Glu Pro Gly Leu Pro Gly Pro Pro Gly
           515                 520                 525
   Phe Pro Gly Ile Gly Lys Pro Gly Val Ala Gly Leu His Gly Pro Pro
       530                 535                 540
   Gly Lys Pro Gly Ala Leu Gly Pro Gln Gly Gln Pro Gly Leu Pro Gly
   545                 550                 555                 560
   Pro Pro Gly Pro Pro Gly Pro Pro Gly Pro Pro Ala Val Met Pro Pro
                   565                 570                 575
   Thr Pro Pro Pro Gln Gly Glu Tyr Leu Pro Asp Met Gly Leu Gly Ile
               580                 585                 590
   Asp Gly Val Lys Pro Pro His Ala Tyr Gly Ala Lys Lys Gly Lys Asn
           595                 600                 605
   Gly Gly Pro Ala Tyr Glu Met Pro Ala Phe Thr Ala Glu Leu Thr Ala
       610                 615                 620
   Pro Phe Pro Pro Val Gly Ala Pro Val Lys Phe Asn Lys Leu Leu Tyr
   625                 630                 635                 640
   Asn Gly Arg Gln Asn Tyr Asn Pro Gln Thr Gly Ile Phe Thr Cys Glu
                   645                 650                 655
   Val Pro Gly Val Tyr Tyr Phe Ala Tyr His Val His Cys Lys Gly Gly
               660                 665                 670
   Asn Val Trp Val Ala Leu Phe Lys Asn Asn Glu Pro Val Met Tyr Thr
           675                 680                 685
   Tyr Asp Glu Tyr Lys Lys Gly Phe Leu Asp Gln Ala Ser Gly Ser Ala
       690                 695                 700
   Val Leu Leu Leu Arg Pro Gly Asp Arg Val Phe Leu Gln Met Pro Ser
   705                 710                 715                 720
   Glu Gln Ala Ala Gly Leu Tyr Ala Gly Gln Tyr Val His Ser Ser Phe
                   725                 730                 735
   Ser Gly Tyr Leu Leu Tyr Pro Met
               740

## Description

The present invention relates to an antibody which binds to an epitope present at the C-terminus of the collagen type VIII α1 chain and to immunoassays detecting said epitope.

Type VIII collagen is a product of endothelial cells, keratinocytes, mast cells, microvascular endothelial cells and some tumour cells. It is also present in a variety of extracellular matrices as diverse as sclera, skin and glomerulus. While the function of collagen type VIII is uncertain recent work has highlighted the importance of this collagen in the vasculature. Particularly significant may be its up-regulation in smooth muscle cell migration and potential role in maintaining the smooth muscle cell phenotype. It is interesting to speculate that this collagen may provide a substratum for a variety of cells and facilitate movement of endothelial cells in angiogenesis, smooth muscle cells in intimal invasion and myofibroblasts in fibrotic conditions [1].

Collagen type VIII is a short chain non-fibrillar collagen and is the major component of the Descemet's membrane (basement membrane separating corneal endothelial cells from corneal stroma) of corneal endothelial cells. It is part of the endothelium of blood vessels and present in arterioles and venules, and thus found in heart, brain, liver, lung, muscle etc., while it is found around the chondrocytes in the cartilage [1]. The human α1 procollagen gene is located on chromosome 3, while the human α2 procollagen gene is located on chromosome 1. Each α chain has a molecular weight of approximately 60kDa [2]. Previously, collagen type VIII has been described as a heterotrimer comprised of two α1 chains and one α2 chain [3], but in vitro studies have shown that homotrimers of either α1 or α2 can also be formed [4]. In addition these homotrimers are pepsin-resistant and an immunohistochemistry study showed they did not always co-localize in the cornea, optic nerve, aorta and umbilical cord [5].

Type VIII collagen is synthesized by aortic and corneal endothelial cells, as well as by pulmonary artery endothelial cells and microvascular endothelial cells. Not all endothelial cells express type VIII collagen, and as such the collagen can be absent from large and small vessels [6]. Human mast cells have also been shown to produce type VIII collagen under normal and pathological conditions, and it has been speculated that this contributes to angiogenesis, tissue remodeling and fibrosis [7].

Angiogenesis, tissue remodeling and fibrosis are important parts of tumor development and progression [8]. The lungs have a large surface area with an associated basement membrane and interstitial matrix, and it is well known that matrix proteins such as type I, III, IV and VI collagen and elastin are elevated in patients with a pulmonary disease [9-13]. Type VIII collagen may be related to cancer since tumor angiogenesis is found in most malignancies. It is indirectly involved in tumorigenic events such as cell proliferation and metastasis due to the dependence on the exchange of oxygen and nutrients with tumor waste products [14]. During angiogenesis, endothelial cells are induced to proliferate and migrate as well as to activate signaling pathways that in turn drive cell shape changes and angiogenic sprouting [15]. Furthermore, the tumor blood vessels often fail to become quiescent due to an altered tissue remodeling leading to sporadic angiogenesis and formation of leaky blood vessels. As with angiogenesis, fibrosis is a phenomenon that can be observed in many malignancies. In cancer fibrosis is also known as desmoplasia. In desmoplasia, an accumulation of perpetually activated cancer associated fibroblasts (CAFs) are observed which exhibit increased and altered expression of extracellular matrix (ECM) proteins including collagens [16]. Desmoplasia is emerging as an important and active process involved in tumor initiation and progression and may, amongst other things, promote the migration of cancer cells [17] .

Korsching et al, [34] discloses and antibody directed against amino acids 100-111 of human *COL8A1* with a kDa about 1.3.nM.

As such, an aim of the present invention is to quantify type VIII collagen in serum samples from patients diagnosed with diseases associated with vascular remodelling and angiogenesis, such as fibrosis and cancer.

The sequence of the human Collagen alpha-1(VIII) is set out in SEQ ID NO. 1. A signal peptide 1-27 (MAVLPGPLQL LGVLLTISLS SIRLIQA) is cleaved off to produce the mature alpha-1 protein chain 28-744. The sequence of the N-terminal of the mature alpha-1 protein chain is therefore NH2-GAYYGIKPLP... and the sequence of the C-terminal is ..SFSGYLLYPM-COOH.

We have now developed a monoclonal antibody and an ELISA kit targeting the C-terminal of the mature Type VIII - α1 chain. We refer to this kit and to reactivity measured with it herein as 'C8-C'.

We have established that levels of C8-C are elevated compared to controls in idiopathic pulmonary fibrosis (IPF), chronic obstructive pulmonary disease (COPD) and squamous cell carcinoma of the lung along with various types of cancer.

The present invention utilises an antibody reactive with a C-terminal epitope of the α1 chain of collagen Type VIII, which specifically binds to a said C-terminal epitope comprised in a C-terminal amino acid sequence ..SFSGYLLYPM-COOH.

The term 'antibody' as used herein includes polyclonal and monoclonal antibodies and also specific binding fragments of antibodies such as Fab or F(ab')2. Thus, said antibody may be a monoclonal antibody or a fragment of a monoclonal antibody having specific binding affinity.

Preferably, said antibody does not recognise or bind an elongated version of said C-terminal amino acid sequence which is ..SFSGYLLYPMA-COOH.

Preferably, said antibody does not recognise or bind (or also does not recognise or bind) a truncated version of said C-terminal amino acid sequence which is ..SFSGYLLYP-COOH.

Preferably, the ratio of the affinity of said antibody for amino acid sequence ..SFSGYLLYPM-COOH to the affinity of said antibody for elongated amino acid sequence ..SFSGYLLYPMA-COOH, and/or to the truncated amino acid sequence ..SFSGYLLYP-COOH, is greater than 10 to 1.

More generally, the ratio of the affinity of said antibody for amino acid sequence ..SFSGYLLYPM-COOH to the affinity of said antibody for said elongated amino acid sequence is preferably greater than 10 to 1, preferably greater than 50 to 1, preferably greater than 100 to 1, preferably greater than 500 to 1, preferably greater than 1000 to 1, and most preferably greater than 10,000 to 1.

Also preferably, the ratio of the affinity of said antibody for amino acid sequence ..SFSGYLLYPM-COOH to the affinity of said antibody for said truncated amino acid sequence is greater than 10 to 1, preferably greater than 50 to 1, preferably greater than 100 to 1, preferably greater than 500 to 1, preferably greater than 1000 to 1, and most preferably greater than 10,000 to 1.

The invention includes a method of immunoassay for detecting or quantitating in a sample a C-terminal epitope of the mature α1 chain of collagen type VIII, wherein said method comprises contacting a sample comprising said terminal epitope with an antibody as described above, and determining the amount of binding of said antibody.

The present invention provides a method of immunoassay for detecting or quantitating in a biofluid sample a C-terminal epitope of the α1 chain of collagen type VIII comprised in the C-terminus amino acid sequence SFSGYLLYPM-COOH, wherein said method comprises contacting said sample comprising said C-terminal epitope with an antibody specifically reactive with an epitope comprised in the C-terminus amino acid sequence SFSGYLLYPM-COOH, and determining the amount of binding of said antibody, wherein said biofluid is serum, or plasma.

Said immunoassay may be a competition assay or a sandwich assay such as a radioimmunoassay or an enzyme-linked immunosorbent assay (ELISA).

Such a method may further comprise correlating the quantity of said C-terminal or N-terminal epitope of the α1 chain of collagen type VIII determined by said method with standard normal values of said C-terminal of the α1 chain of collagen type VIII to evaluate a change thereof from normal levels.

This biomarker may be used to assist in the diagnosis of disease states, or to provide prognosis as to which patients are likely to suffer more rapid deterioration of their condition, which may make them more relevant patients to take into a clinical trial of a relevant treatment. Such disease states and/or conditions include fibrosis and cancer. Fibrotic conditions include (but are not limited to) idiopathic pulmonary fibrosis (IPF) and chronic obstructive pulmonary disease (COPD), and cancers include (but are not limited to) breast cancer, colon cancer, melanoma, non-squamous cell carcinoma of the lung (NSCLC), ovarian cancer, pancreatic cancer, prostate cancer, and squamous cell carcinoma of the lung (SCLC).

A further aspect of the disclosure provides an assay kit for determining the quantity of a C-terminal epitope of the α1 chain of collagen Type VIII, comprising an immunological binding partner of the invention and at least one of:
- a streptavidin coated 96 well plate
- a peptide which is reactive with said immunological binding partner, which may be a biotinylated peptide Biotin-L-SFSGYLLYPM-COOH, wherein L is an optional linker
- an optionally biotinylated secondary antibody for use in a sandwich immunoassay
- a calibrator peptide comprising the C-terminal sequence ..SFSGYLLYPM-COOH
- an antibody HRP labelling kit
- an antibody radiolabeling kit
- an assay visualization kit

The invention will be further described and illustrated with reference to the accompanying drawings in which:

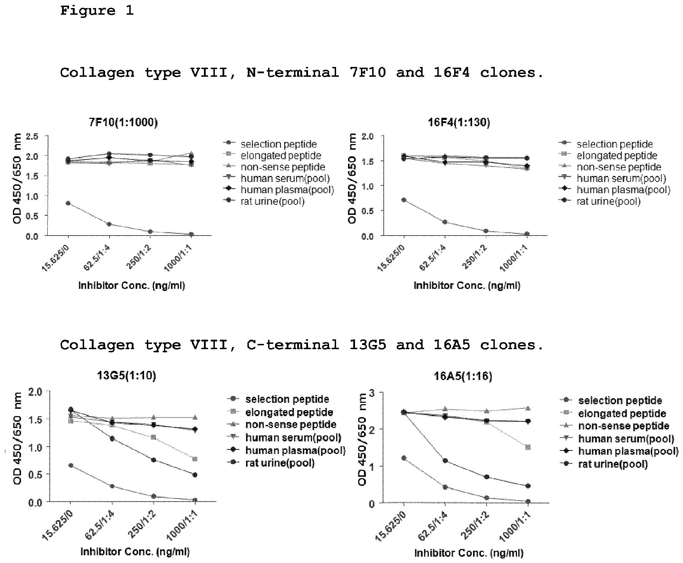

Figure 1 shows results from a peptide specificity test of two monoclonal antibodies;
Figure 2 shows results from a test of the reactivity of monoclonal antibody 13G5 in human serum;
Figure 3 shows results from a further test of the reactivity of monoclonal antibody 13G5 in human serum; and
Figure 4 shows results from a further test of the reactivity of monoclonal antibody 13G5 in human serum.

### Examples

### Example 1: Antibody development for C8-C assay, clone 13G5;

We used the last 10 amino acids of the type VIII collagen α1 chain (^{735'}SFSGYLLYPM^{'744}) as an immunogenic peptide to generate specific epitope monoclonal antibodies. The methods used for monoclonal antibody development were as previously described [18]. Briefly, 4-6-week-old Balb/C mice were immunized subcutaneously with 200µl emulsified antigen with 50µg of the immunogenic peptide. Consecutive immunizations were performed at 2-week intervals in Freund's incomplete adjuvant, until stable sera titer levels were reached, and the mice were bled from the 2nd immunization on. At each bleeding, the serum titer was detected and the mouse with highest antiserum titer and the best native reactivity was selected for fusion. The selected mouse was rested for 1 month followed by intravenous boosting with 50µg of immunogenic peptide in 100µl 0.9% sodium chloride solution 3 days before isolation of the spleen for cell fusion.

The fusion procedure has been described elsewhere [19]. Briefly, mouse spleen cells were fused with SP2/0 myeloma fusion partner cells. The fusion cells were raised in 96-well plates and incubated in the CO2-incubator. Here standard limited dilution was used to promote monoclonal growth. Cell lines specific to the selection peptide and without cross-reactivity to elongated peptide (SFSGYLLYPMA, Chinese Peptide Company, China) were selected and sub-cloned. At last the antibodies were purified using an IgG column.

### C8-C assay protocol:

ELISA-plates used for the assay development were Streptavidin-coated from Roche (cat.: 11940279). All ELISA plates were analyzed with the ELISA reader from Molecular Devices, SpectraMax M, (CA, USA). We labelled the selected monoclonal antibody with horseradish peroxidase (HRP) using the Lightning link HRP labelling kit according to the instructions of the manufacturer (Innovabioscience, Babraham, Cambridge, UK). A 96-well streptavidin plate was coated with biotinylated synthetic peptide biotin-KKKSFSGYLLYPM (Chinese Peptide Company, China) dissolved in assay buffer (50 mM Trizma, 0.46 mM Tween 20, 0.08 mM Phenol Red, 34 mM NaCl, 0.36% Bronidox L5, 1% BSA, pH 7.4) and incubated 30 minutes at 20°C. 20 µL of standard peptide or samples diluted in assay buffer were added to appropriate wells, followed by 100 µL of HRP conjugated monoclonal antibody 13G5, and incubated 20 hour at 4°C. Finally, 100 µL tetramethylbenzinidine (TMB) (Kem-En-Tec cat.4380H) was added and the plate was incubated 15 minutes at 20°C in the dark. All the above incubation steps included shaking at 300 rpm. After each incubation step the plate was washed five times in washing buffer (20 mM Tris, 50 mM NaCl). The TMB reaction was stopped by adding 100 µL of stopping solution (1% H₂SO₄) and measured at 450 nm with 650 nm as the reference.

### C8-C technical evaluation:

The lowest limit of detection (LLOD) was determined from 21 zero samples (i.e. buffer) and calculated as the mean + 3x standard deviation. The intra-assay variation and inter-assay variations were determined by 12 independent runs of 8 QC samples with each run consisting of double determinations of the samples. Dilution recovery was determined in 4 serum samples and 4 EDTA plasma samples and was calculated as a percentage of recovery of diluted samples from the 100% sample. The data for the technical validation is seen in Table 1.

**Table 1. Technical validation of the C8-C assay.**

| | |
|---|---|
| Detection range | 0.37 nM - 111 nM |
| Lower limit of quantification | 3.4 nM |
| Intra-assay variation | 6% |
| Inter-assay variation | 12.70% |
| Dilution range of serum samples | 1:2 (recommended |
| Dilution range of plasma samples (EDTA) | 1:2 (recommended |
| Dilution recovery in serum | 97.50% |
| Dilution recovery in plasma (EDTA) | 91% |
| Analyte stability serum (24 h, 4C/20C) | 94%/67.3% |
| Analyte stability plasma (EDTA) (24 h, 4C/20C) | 83.3%/74.4% |

### Example 2:

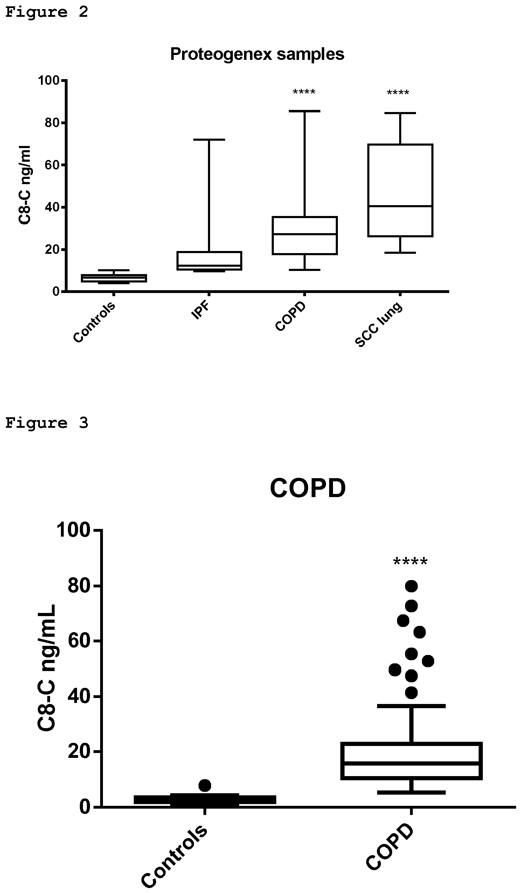

Using monoclonal antibody 13G5 C8-C was measured in serum samples from patients diagnosed with COPD (n = 13), IPF (n = 10) and squamous cell carcinoma lung cancer (n = 10) obtained from Proteogenex (Culver City, CA) and compared to non-diseased controls. Results are shown in Figure 2.

Results are shown as mean ± standard error of mean (SEM). Differences between mean values were compared by nonparametric Kruskal-Wallis one-way ANOVA test. All statistical analyses were performed in GraphPad Prism software v.6 (GraphPad Software, San Diego, CA). P values less than 0.05 were considered significant.

Levels of C8-C were found to be elevated in all these clinical conditions.

### Example 3:

C8-C was evaluated in a larger COPD cohort. The concentrations of C8-C were measured in serum samples from patients diagnosed with COPD (n = 68) obtained from Hvidovre Hospital (Hvidovre Hospital, Denmark) and compared to non-diseased controls (n = 20).

Figure 3 shows that the concentration of C8-C was significantly elevated in the patients diagnosed with COPD when compared to controls (p<0.0001).

### Example 4:

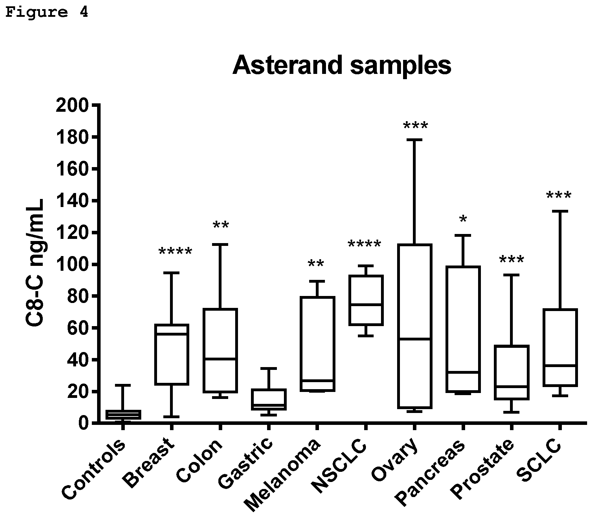

Using monoclonal antibody 13G5 C8-C levels were measured in serum samples from patients diagnosed with breast (n = 13), colon (n = 7), gastric (n = 9), melanoma (n =7), NSCLS (n =12), ovary (n = 10), pancreas (n = 5), prostate (n = 14) and SCLC cancer (n = 8) obtained from Asterand (Detroit, MI) and compared to non-diseased controls (n = 43). The samples were diluted 1:2 in the C8-C assay. Results are shown in Figure 4.

Levels of C8-C were found to be elevated in all these clinical conditions.

### Discussion

To our knowledge this is the first disclosure describing the development and validation of a novel competitive ELISA for the assessment of a monoclonal antibody directed against the C-terminal of type VIII collagen. The assay was shown to be technically robust, showing low values of LLOD, intra- and inter-assay variation and interference with an acceptable dilution recovery and analyte stability. The sequence alignment of human, rat, mouse and bovine C-terminal of type VIII collagen shows 100% homology between the species. The monoclonal antibody 13G5 is specific towards the C-terminal sequence SFSGYLLYPM. The assay did not detect the elongated (one additional amino acid) or a nonsense peptide indicating that the monoclonal antibody is specific towards the C-terminal of type VIII collagen. The C8-C competitive ELISA works for assessments in both human and rodent matrices, which allow good translational science.

The presently described C8-C assay is different to other commercially available assays since other commercial type VIII collagen assays available utilize either monoclonal or polyclonal antibodies for which the precise epitope is not known. Kapoor et al. raised both polyclonal and monoclonal antibodies towards triple-helical domains of type VIII collagen from 50-kD fragments derived from Descemet's membrane [20,21]. The fragments used for immunization are pepsin-resistant but not resistant towards collagenases [21]. Within tissues there is constant ongoing remodelling, with a fine balance between formation and degradation of the proteins. In diseases, such as fibrosis or cancer, there is an increased tissue turnover and the balance is shifted towards formation leading to a net increase of matrix proteins, but it is important to note that the degradation of matrix proteins is also increased. Given this, it is highly plausible that type VIII collagen is cleaved by collagenases during tissue remodelling. The NB683-13G5 antibody deployed in the C8-C assay has the advantage that it is specific to a small sequence consisting of only ten amino acids, which ensures that it detects even the small fragments generated by protease degradation. The antibodies generated by Kapoor et al. and Sawada et al. recognize a fairly large peptide that is likely to be degraded [21,22], hence a smaller pool of type VIII collagen is detected.

Type VIII collagen has been shown to be elevated following vascular injury and is part of the tissue remodeling which takes place following injury [23]. In addition type VIII collagen has been localized to endothelial cells during sprouting and when endothelial cells are exposed to angiogenic factors a 4-6-fold increase of type VIII collagen can be seen [24]. Gene expression of type VIII collagen is known to be elevated in tumor-associated stroma and has been shown to be elevated in hepatocellular carcinoma cells [25]. To our knowledge we are the first to show that concentrations of type VIII collagen is elevated in the circulation of patients diagnosed with COPD and SCC lung cancer. It is generally accepted that vascular endothelial growth factor (VEGF) is upregulated in COPD, while it is well known that vascular remodeling takes place. Recently, it has been accepted that angiogenesis is increased in patients with COPD [26,27]. In order to confirm the elevated concentrations of C8-C observed for COPD patients, a larger cohort of COPD patients was assessed. It was found that significantly increased concentrations of C8-C in serum from COPD patients can be seen when compared to controls (p < 0.0001). More precisely, a 7-fold increase of C8-C concentrations was seen in serum from COPD patients compared to controls.

In addition the concentrations of type VIII collagen were significantly elevated in patients diagnosed with breast-, colon-, melanoma-, NSCLC-, ovary-, pancreas-, prostate- and SCLC cancer, but not gastric cancer. In short, increases between 5-12-fold were seen within various cancer types, except for gastric cancer. Expression of type VIII collagen has previously been shown to be elevated in the blood vessels of some types of brain tumors, and several carcinomas [28]. In addition type VIII collagen is expressed by endothelial cells and smooth muscle cells, especially after vascular injury, and by tumor cells. Integrin α2β1 is a known receptor for collagen type I-VIII [29], which studies have shown to be increased in metastatic cells when compared to cells in the primary tumor [30]. The receptor is associated with tumor progression and invasion in several cancer types. Type VIII collagen is able to regulate smooth muscle cell (SMC) migration through the β1 receptor [29], with SMCs being important players in fibrotic diseases and the tumor microenvironment [31]. Gastric cancer was the only cancer form not showing increased concentrations of C8-C in serum. The literature states that there is ECM remodeling ongoing in gastric cancer tissue [32], but these data are based on unspecified collagen metabolism or type I, III and IV collagen. The inventors of the present invention were not able to find any literature confirming the presence of type VIII collagen in the stomach or in gastric related cancers, which could explain the low concentrations of C8-C in the serum of patients diagnosed with gastric cancer.

In conclusion the inventors of the present invention are the first to develop a technically robust type VIII collagen assay and show that the concentrations of type VIII collagen are significantly elevated in serum from patients diagnosed with COPD and various types of cancer. The antibody NB683-13G5 was generated and implemented in the C8-C competitive ELISA and found specific for the C-terminal part of type VIII collagen.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'.

### References

1. Kittelberger, R., Davis, P. F., Flynn, D. W. & Greenhill, N. S. Distribution of type VIII collagen in tissues: an immunohistochemical study. Connect. Tissue Res. 24, 303-318 (1990) .
2. Ma, Z.-H., Ma, J.-H., Jia, L. & Zhao, Y.-F. Effect of enhanced expression of COL8A1 on lymphatic metastasis of hepatocellular carcinoma in mice. Exp. Ther. Med. 4, 621-626 (2012).
3. Jander, R., Korsching, E. & Rauterberg, J. Characteristics and in vivo occurrence of type VIII collagen. Eur. J. Biochem. 189, 601-607 (1990).
4. Illidge, C., Kielty, C. & Shuttleworth, A. The alphal(VIII) and alpha2(VIII) chains of type VIII collagen can form stable homotrimeric molecules. J. Biol. Chem. 273, 22091-5 (1998).
5. Greenhill, N. S., Rüger, B. M., Hasan, Q. & Davis, P. F. The alphal(VIII) and alpha2(VIII) collagen chains form two distinct homotrimeric proteins in vivo. Matrix Biol. 19, 19-28 (2000) .
6. Sage H, Balian G, Vogel AM, Bornstein P. Type VIII collagen. Synthesis by normal and malignant cells in culture. Lab Invest 1984;50:219-31.
7. Rüger B, Dunbar PR, Hasan Q, Sawada H, Kittelberger R, Greenhill N, et al. Human mast cells produce type VIII collagen in vivo. Int J Exp Pathol 1994;75:397-404.
8. Lu P. Extracellular Matrix Degradation and Remodeling in Development and Disease. Cold Spring Harb Perspect Biol 2011;3.
9. Sand JMB, Knox AJ, Lange P, Sun S, Kristensen JH, Leeming DJ, et al. Accelerated extracellular matrix turnover during exacerbations of COPD. Respir Res 2015;16:69. doi:10.1186/sl2931-015-0225-3.
10. Ricard-Blum S, Dublet B, Rest M van der. Unconventional Collagens: Types VI, VII, VIII, IX, X, XII, XIV, XVI, and XIX. Oxford University Press; 2000.
11. Sand JM, Genovese F, Martinez F, Han M, Hogaboam C, Karsdal MA, et al. Serum levels of a MMP-12 generated type IV collagen fragment is elevated in COPD and IPF patients. Eur Respir J 2013;42:P838 - .
12. Willumsen N, Bager CL, Leeming DJ, Smith V, Christiansen C, Karsdal MA, et al. Serum biomarkers reflecting specific tumor tissue remodeling processes are valuable diagnostic tools for lung cancer. Cancer Med 2014;3:1136-45. doi:10.1002/cam4.303.
13. Kristensen JH, Larsen L, Dasgupta B, Brodmerkel C, Curran M, Karsdal MA, et al. Levels of circulating MMP-7 degraded elastin are elevated in pulmonary disorders. Clin Biochem 2015. doi:10.1016/j.clinbiochem.2015.07.009.
14. Nishida N, Yano H, Nishida T, Kamura T, Kojiro M. Angiogenesis in cancer. Vasc Health Risk Manag 2006;2:213-9.
15. Chung, AS. FN. The Extracellular Matrix & Angiogenesis: Role of the Extracellular Matrix in Developing Vessels and Tumor Angiogenesis. Pathways 2010:2-5.
16. Kalluri R, Zeisberg M. Fibroblasts in cancer. Nat Rev Cancer 2006;6:392-401. doi:10.1038/nrc1877.
17. Egeblad M, Rasch MG, Weaver VM. Dynamic interplay between the collagen scaffold and tumor evolution. Curr Opin Cell Biol 2010;22:697-706. doi:10.1016/j.ceb.2010.08.015.
18. Barascuk, N. et al. A novel assay for extracellular matrix remodeling associated with liver fibrosis: An enzyme-linked immunosorbent assay (ELISA) for a MMP-9 proteolytically revealed neo-epitope of type III collagen. Clin. Biochem. 43, 899-904 (2010).
19. Gefter, M. L., Margulies, D. H. & Scharff, M. D. A simple method for polyethylene glycol-promoted hybridization of mouse myeloma cells. Somatic Cell Genet. 3, 231-6 (1977).
20. Kapoor R, Bornstein P, Sage EH. Type VIII collagen from bovine Descemet's membrane: structural characterization of a triple-helical domain. Biochemistry 1986;25:3930-7.
21. Kapoor R, Sakai LY, Funk S, Roux E, Bornstein P, Sage EH. Type VIII collagen has a restricted distribution in specialized extracellular matrices. J Cell Biol 1988;107:721-30. doi:10.1083/jcb.107.2.721.
22. Sawada H, Konomi H, Hirosawa K. Characterization of the collagen in the hexagonal lattice of Descemet's membrane: Its relation to type VIII collagen. J Cell Biol 1990;110:219-27. doi:10.1083/jcb.110.1.219.
23. Leena Karttunen, Ute Felbor, Miikka Vikkula BRO. Vascular Matrix and Disorders. In: Zon LI, editor. Hematop. A Dev. Approach, New York: Oxford University Press, USA; 2001, p. 784-95.
24. Israel Vlodavsky GC. Fibroblast Growth Factors in Tumor Progression and Angiogenesis. In: Teicher BA, editor. Antiangiogenic Agents Cancer Ther., New York: Springer Science & Business Media; 1998, p. 93-119.
25. Wang W, Xu G, Ding C-L, Zhao L-J, Zhao P, Ren H, et al. All-trans retinoic acid protects hepatocellular carcinoma cells against serum-starvation-induced cell death by upregulating collagen 8A2. FEBS J 2013;280:1308-19. doi:10.1111/febs.12122.
26. Matarese A, Santulli G. Angiogenesis in Chronic Obstructive Pulmonary Disease 2012. doi:10.1038/npre.2012.7112.1.
27. Harkness LM, Kanabar V, Sharma HS, Westergren-Thorsson G, Larsson-Callerfelt A-K. Pulmonary vascular changes in asthma and COPD. Pulm Pharmacol Ther 2014;29:144-55. doi:10.1016/j.pupt.2014.09.003.
28. Paulus W, Sage EH, Jellinger K, Roggendorf W. Type VIII collagen in the normal and diseased human brain. Acta Histochem Suppl 1992;42:195-9.
29. Adiguzel E, Hou G, Sabatini PJB, Bendeck MP. Type VIII collagen signals via β1 integrin and RhoA to regulate MMP-2 expression and smooth muscle cell migration. Matrix Biol 2013;32:332-41. doi:10.1016/j.matbio.2013.03.004.
30. Klein CE, Dressel D, Steinmayer T, Mauch C, Eckes B, Krieg T, et al. Integrin alpha 2 beta 1 is upregulated in fibroblasts and highly aggressive melanoma cells in three-dimensional collagen lattices and mediates the reorganization of collagen I fibrils. J Cell Biol 1991;115:1427-36.
31. Sridhara SU, Choudaha N, Kasetty S, Joshi PS, Kallianpur S, Tijare M. Stromal myofibroblasts in nonmetastatic and metastatic oral squamous cell carcinoma: An immunohistochemical study. J Oral Maxillofac Pathol 2013;17:190-4. doi:10.4103/0973-029X.119758.
32. Yamaguchi H, Yoshida N, Takanashi M, Ito Y, Fukami K, Yanagihara K, et al. Stromal fibroblasts mediate extracellular matrix remodeling and invasion of scirrhous gastric carcinoma cells. PLoS One 2014;9:e85485. doi:10.1371/journal.pone.0085485.
33. Korsching E and Rauterberg J. Generation of type VIII collagen specific antibodies. J. Immunological Methods, 1995; 188: 521-62.

## Claims

1. A method of immunoassay for detecting or quantitating in a biofluid sample a C-terminal epitope of the α1 chain of collagen type VIII comprised in the C-terminus amino acid sequence SFSGYLLYPM-COOH, wherein said method comprises contacting said sample comprising said C-terminal epitope with an antibody specifically reactive with an epitope comprised in the C-terminus amino acid sequence SFSGYLLYPM-COOH, and determining the amount of binding of said antibody, wherein said biofluid is serum, or plasma.

2. A method as claimed in claim 1, wherein said immunoassay is a competition assay or a sandwich assay.

3. A method as claimed in claim 2, wherein said immunoassay is a radioimmunoassay or an enzyme-linked immunosorbent assay.

4. A method as claimed in any one of claims 1 to 3, further comprising correlating the quantity of said C-terminal epitope of the α1 chain of collagen type VIII determined by said method with non-diseased control values of said C-terminal epitope of the α1 chain of collagen type VIII to evaluate a change thereof from non-diseased levels.

5. A method as claimed in any preceding claim, wherein the antibody is a monoclonal antibody.

## Patentansprüche

1. Verfahren eines Immunassays, um in einer Probe einer biologischen Flüssigkeit ein C-terminales Epitop der α1-Kette von Kollagen Typ III nachzuweisen oder zu quantifizieren, das die C-terminale-Aminosäuresequenz SFSGYLLYPM-COOH umfasst, wobei das Verfahren das Kontaktieren der Probe, die das C-terminale Epitop umfasst, mit einem Antikörper umfasst, der besonders reaktiv mit einem Epitop reagiert, das in der C-terminalen Aminosäuresequenz SFSGYLLYPM-COOH enthalten ist, und Bestimmen des Betrags der Bindung des Antikörpers, wobei die biologische Flüssigkeit Serum oder Plasma ist.

2. Verfahren nach Anspruch 1, wobei das Immunassay ein kompetitives Assay oder ein Sandwich-Assay ist.

3. Verfahren nach Anspruch 2, wobei das Immunassay ein Radioimmunassay oder ein Enzymimmunassay ist.

4. Verfahren nach einem der Ansprüche 1 bis 3, ferner umfassend das Korrelieren der Quantität des C-terminalen Epitops der α1-Kette von Kollagen Typ III, die mit dem Verfahren bestimmt wurde, mit gesunden Kontrollwerten des C-terminalen Epitops der α1-Kette von Kollagen Typ III, um eine Veränderung davon aus gesunden Spiegeln zu evaluieren.

5. Verfahren nach einem vorhergehenden Anspruch, wobei der Antikörper ein monoklonaler Antikörper ist.

## Revendications

1. Procédé de dosage immunologique permettant de détecter ou de quantifier dans un échantillon de biofluide un épitope C-terminal de la chaîne α1 du collagène de type VIII compris dans la séquence d'acides aminés C-terminale SFSGYLLYPM-COOH, ledit procédé comprenant la mise en contact dudit échantillon comprenant ledit épitope C-terminal avec un anticorps spécifiquement réactif avec un épitope compris dans la séquence d'acides aminés C-terminale SFSGYLLYPM-COOH, et la détermination de la quantité de liaison dudit anticorps, ledit biofluide étant du sérum ou du plasma.

2. Procédé selon la revendication 1, dans lequel ledit dosage immunologique est un essai de compétition ou un essai de type sandwich.

3. Procédé selon la revendication 2, dans lequel ledit dosage immunologique est un radioimmunodosage ou une méthode immuno-enzymatique ELISA.

4. Procédé selon l'une quelconque des revendications 1 à 3, comprenant en outre la corrélation de la quantité dudit épitope C-terminal de la chaîne α1 du collagène de type VIII déterminée par ledit procédé avec des valeurs témoins non malades dudit épitope C-terminal de la chaîne α1 du collagène de type VIII pour évaluer un changement de celui-ci par rapport aux niveaux non malades.

5. Procédé selon une quelconque revendication précédente, dans lequel l'anticorps est un anticorps monoclonal.
